# EUROPÄISCHE PATENTSCHRIFT

(11) **EP 2 275 524 B1**
(45) Veröffentlichungstag und Bekanntmachung des Hinweises auf die Patenterteilung: **12.10.2011**
(21) Anmeldenummer: 10010282.1
(22) Anmeldetag: 09.02.2009
(51) Int. Cl.: C11D 1/00, C11D 3/37, C11D 17/00

(54) **Haftendes Mittel zur Applikation auf einem Sanitärgegenstand**
Adhesive agent for application on a sanitary object
Agent adhésif pour l'application à l'object sanitaire

(30) Priorität: 29.02.2008 DE 102008012092; 14.10.2008 DE 102008051173
(43) Veröffentlichungstag der Anmeldung: 19.01.2011
(62) Teilanmeldung aus: 09714305.1
(73) Patentinhaber: Buck-Chemie GmbH, 71083 Herrenberg (DE)
(72) Erfinder: Leipold, Joachim, Dr., 72760 Reutlingen (DE); Fritz, Matthias, 72810 Gomaringen (DE); Jaeschke, Edgar, 70794 Filderstadt (DE)
(74) Vertreter: Mammel, Ulrike

(56) Entgegenhaltungen:
- EP-A- 0 850 649
- WO-A-99/66021
- WO-A-03/035511
- DE-A1- 10 048 887
- DATABASE WPI Week 200206 Thomson Scientific, London, GB; AN 2002-045086 XP002529049, & JP 2001 270996 A (NAKAMURA A) 2. Oktober 2001 (2001-10-02)
- Technical Bulletin Mazol 31K from BASF Corporation
- PALL CORPORATION: 'Handbuch', Seite 29
- Kraton Sicherheitsdatenblatt
- ESCOREZ 5000 Series of Exxon Mobil, Safety data sheet
- IRGANOX 1010 data sheet of Ciba Specialty Chemicals
- WIKIPEDIA : Seife, Magnesiumstearat, Kalkseife

## Beschreibung

Die vorliegende Erfindung betrifft ein haftendes Mittel für den Sanitärbereich, das insbesondere zur Applikation auf einen Sanitärgegenstand wie einem Toilettenspülbecken dient.

Diese Mittel sind viskose, im Allgemeinen pastöse Mittel, die z.B. aus einem entsprechenden Behältnis direkt auf die Oberfläche des Sanitärgegenstandes aufgebracht werden, dort haften und erst nach einer größeren Anzahl von Spülvorgängen abspülbar sind.

Aus der WO 99/66017 sind haftende Sanitärmittel bekannt, die zur Reinigung und Beduftung dienen und Tenside, Wasser, Duftstoffe und Haftvermittler umfassen. Nach der direkten Applikation auf dem Sanitärgegenstand werden diese Sanitärmittel erst nach einer größeren Anzahl von Spülzyklen abgespült.

Eine Weiterentwicklung dieser haftenden Sanitärmittel mit glatteren Oberflächen infolge von Zusätzen von mehrwertigen Alkoholen ist in der EP 1 325 103 B1 offenbart.

Weitere haftende Sanitärmittel auf der Grundlage der Oligo- oder Polyalkylenoxide umfassenden Blockcopolymeren oder der Aryl-Ethoxylate oder Alkyl-Arylethoxylate als Haftvermittler sind in der EP 1 318 191 B1 beschrieben und haftende Sanitärmittel mit Bleichmitteln in der DE 10 2004 056 554 A1.

Die bekannten haftenden Sanitärmittel lassen sich auf einfache und hygienische Art und Weise mit einer geeigneten Vorrichtung applizieren, sie haften an der Oberfläche des Sanitärgegenstands, behalten ihre Form bei und werden selbst unter Einwirkung von Wasser nicht als Ganzes herabgespült, sondern erst nach einer Vielzahl von Spülungen nach und nach vollständig aufgelöst.

Der besondere Vorteil dieser haftenden Sanitärmittel besteht darin, dass zusätzliche Behältnisse wie die so genannten "WC-Körbchen", deren Benutzung vom Verbraucher insbesondere beim Ersetzen des Sanitärmittels und bei der Reinigung der Toilette als unhygienisch empfunden wird, vermieden werden.

Auf dem Gebiet der herkömmlichen stückförmigen Toilettenreinigungs- und Beduftungsmittel, die in WC-Körbchen in der Toilettenschüssel eingesetzt werden, wurde in den letzten Jahren eine Vielzahl von Mehrphasenprodukten angeboten:
So sind beispielsweise Mittel mit einer zusätzlichen Bleichmittelphase in der WO 00/23558 beschrieben, Mittel mit einer Reinigungsmittelformkörperphase und einer Gelphase in der EP 1 418 225 A1 und Reinigungsmittelformkörper mit einer wasserlösliche Salze umfassenden und einer wasserunlösliche Salze umfassenden Phase in der WO 00/58434.

Solche mehrphasigen Mittel können durch die Aufteilung der verschiedenen von einem Toilettenreinigungs- und Beduftungsmittel zu erfüllenden Funktionen auf mehrere Phasen, beispielsweise einer speziellen Phase für die intensive Beduftung und einer speziellen Phase ausschließlich für die Reinigung, die Anforderungen der Verbraucher besser erfüllen.

Weiterhin sind aus der WO 03/035511 A1 thixotrope Mittel für den Haushalt, die Versagel 1600, Mineralöl, Silikon und Monooleat enthalten, bekannt und aus der EP 0 850 649 A1 Klebemittel für Kosmetikzwecke enthaltend Styrol-Block-Copolymere oder hydrierte Styrol-Block-Copolymere.

Der vorliegenden Erfindung liegt die Aufgabe zugrunde, ein Sanitärmittel bereitzustellen, das auf einfache und hygienische Art und Weise applizierbar ist, das vielseitig einsetzbar ist und das den Verbraucherwünschen in Bezug auf intensive Beduftung und gute Reinigung gerecht wird.

Diese Aufgabe wird durch die Merkmale des Anspruchs 1 gelöst.

Erstaunlicherweise wurde festgestellt, dass ein Mittel mit einer Viskosität von wenigstens 30 Pa s, gemessen mit einem Haake-Viskosimeter, System Platte/Platte, Plattendurchmesser 10 mm, bei einem Schergefälle von 2,62 s⁻¹ und 20 °C, das Füllstoffe enthält sowie einen Haftvermittler, wobei der Haftvermittler ausgewählt ist aus der Gruppe der hydrierten Polystyrolderivate, der Olefinhomopolymere und der Copolymere von zwei oder mehr Olefinen, wobei die Olefinhomopolymere und - copolymere auch teilhydriert sein können, nicht nur unmittelbar auf den Sanitärgegenstand aufbringbar ist, dort haftet und erst nach einer größeren Anzahl von Spülvorgängen abspülbar ist, sondern infolge seiner Klebrigkeit auch zur Befestigung von stückförmigen Mitteln an der Toilettenschüssel dienen kann.

Die speziellen Haftvermittler bewirken, dass das Mittel auf der Sanitäroberfläche haftet und dass weitere Materialien wie stückförmige Mittel zum Beispiel mit reinigenden oder duftenden Wirkstoffen an der Oberfläche des Haftmittels festgeklebt werden können. Diese Haftvermittler zeichnen sich zudem dadurch aus, dass sich die diese Haftvermittler umfassenden Mittel rückstandslos abspülen lassen und dass die diese Haftvermittler umfassenden Mittel unempfindlich gegenüber Schwankungen im Mischungsverhältnis Haftvermittler zu Füllstoff sind.

Durch den Zusatz von geeigneten Füllstoffen kann beispielsweise die Viskosität und/oder die Abspülbarkeit des Mittels eingestellt und die gewünschte Beduftung oder Einfärbung des Mittels erreicht werden.

Durch das erfindungsgemäße Mittel wird ein breiter Anwendungsbereich eröffnet:
In der Anwendung des Mittels als "reiner Klebstoff" dient das Mittel als Ersatz für die vom Verbraucher als unhygienisch empfundene WC-Körbchen. An dem auf der Toilettenschüsseloberfläche applizierten Haftmittel können Wirkstoffe enthaltende stückförmige Mittel mit eigenen Funktionalitäten wie herkömmliche Rimblocks mit einer oder mehreren Phasen, Rimblocks mit Duftphase, Beduftungstabletten, Rimblocks mit Bleichmittel etc. befestigt werden, wobei das Haftmittel in hygienischer Weise das WC-Körbchen ersetzt und nach und nach vom Spülwasserstrom zusammen mit dem daran klebenden stückförmigen Mittel abgespült wird. Selbstverständlich können an dem Haftmittel auch aus Pulvern oder Granulaten gepresste Tabletten oder auch Wirkstoffe enthaltende, wasserlösliche oder wasserunlösliche Kunststoffe befestigt werden.

In dieser Anwendung weist das Mittel wenigstens einen der erfindungsgemäßen Haftvermittler und einen Verdicker auf. Nachdem die erfindungsgemäßen Haftvermittler im Allgemeinen viskose Flüssigkeiten sind, ist die Zugabe eines Verdickers als Füllstoff erforderlich, damit das Mittel die gewünschte hohe Viskosität von wenigstens 30 Pa s aufweist.

Selbstverständlich können dieser Grundrezeptur aus Haftvermittler und Verdicker noch weitere Bestandteile wie Farb-, Duftstoffe, ggf. Tenside, Schäumer etc. zugegeben werden.

In einer Abwandlung der ersten Ausführungsform weist das Mittel neben dem Haftvermittler und Verdicker auch Parfüm auf, so dass es gleichzeitig zum Ankleben und zur Beduftung eingesetzt werden kann.

In der zweiten Anwendung weist das Haftmittel die erfindungsgemäßen Haftvermittler und als Füllstoff Tenside auf, so dass es durch die geeignete Auswahl der Art und Konzentration der Tenside und ggf. weiterer Zusätze selbst die Reinigungsfunktion übernehmen kann. Durch die Klebewirkung ist die Anbringung weiterer erwünschter Wirkstoffmittel in der Tollettenschüssel, z.B. eines Duftmittels, einer Bleichmitteltablette, einer Entkalkungstablette, eines Werbeträgers etc. ermöglicht.

Mit dem reinigenden Haftmittel kann der Verbraucher somit beispielsweise einen seinen momentanen Wünschen entsprechenden Duft auswählen und die entsprechende Beduftungstablette an das aufgetragene reinigende Haftmittel andrücken, so dass sie auf dem Haftmittel festklebt, beispielsweise, um die Toilette für die nächsten 1 oder 2 Wochen zu beduften.

Das erfindungsgemäße Haftmittel ermöglicht somit sogar, das Haftmittel zusammen mit einer Reihe von Beduftungstabletten oder Beduftungsgelen, Bleichmitteltabletten, Entkalkungstabletten, Intensivreinigungstabletten etc. in einem gemeinsamen Set anzubieten und dem Benutzer somit eine individuelle Bestückung des Haftmittels zu ermöglichen.

Wünscht der Verbraucher eine zusätzliche Bleiche, so wird zum Beispiel eine Bleichtablette auf der Oberfläche des Haftmittels aufgeklebt.

Wünscht der Verbraucher eine besonders starke und intensive Reinigung der Toilette, so kann zusätzlich auch noch eine Reinigungstablette auf dem reinigenden Haftmittel aufgeklebt werden. Auch der Aufbau von mehrschichtigen Mitteln bestehend aus den Schichten Kleber - Funktionalität - Kleber - Funktionalität ist möglich.

Im Gegensatz dazu zeigen die anfangs beschriebenen bislang bekannten Mittel zwar eine gute und andauernde Haftung auf dem Sanitärgegenstand, ein "Ankleben" eines anderen Mittels ist jedoch mit den bislang bekannten haftenden Sanitärmitteln nicht möglich.

Neben dem Einsatz auf dem Gebiet der Toilettenhygiene kann das Mittel auch als Kaltkleber eingesetzt werden, der infolge der zugesetzten Tenside mit Wasser abspülbar ist. Das Mittel kann als Kaltkleber im Sanitärbereich z.B. zum Befestigen von Gegenständen in Urinalen, Pissoirs, aber auch an Handspülbecken oder an Kacheln in der Dusche eingesetzt werden oder auch in Küchen, Restaurants, Schlachthäusern oder anderen Orten, an denen mit Wasser nachgewaschen wird.

Durch den Einsatz des Kaltklebers an Orten, an denen mit Wasser nachgespült wird, erfolgt zusammen mit dem Abspülen des Haftmittels gleichzeitig auch eine Reinigung durch die in dem Haftmittel enthaltenen Tenside.

Ebenso denkbar ist der Einsatz des erfindungsgemäßen Mittels in Waschanlagen für die Reinigung von Kraftfahrzeugen oder zur Applikation an/in Abflüssen oder Gullis, beispielsweise, um diese temporär zu beduften.

Auch als abwaschbare Paste zur Aufnahme von Ködern gegen Ungeziefer oder zur Befestigung an Scheiben oder Fassaden, die beregnet werden, so dass sich das Mittel sukzessive verbraucht, ist das erfindungsgemäße Mittel einsetzbar.

Die einzelnen Bestandteile des erfindungsgemäßen Mittels werden nachfolgend beschrieben:
Bei den Haftvermittlern aus der Gruppe der Polystyrolderivate handelt es sich vorzugsweise um in Mineralöl gelöste, quervernetzte Polystyrolderivate, insbesondere um Alkylenstyrol-Copolymere, wie beispielsweise den hydrierten Butylen/Ethylen/Styrol-Copolymeren und den hydrierten Ethylen/Propylen/Styrol-Copolymeren, die beispielsweise von der Firma Penreco unter dem Handelsnamen Versagel M750 oder Versagel M1600 erhältlich sind.

Weiterhin können als Haftvermittler nicht wasserlösliche Olefinhomopolymere und Copolymere von zwei und mehr Olefinen eingesetzt werden. Zu diesen Verbindungen zählen z.B. die Polybutadienkautschuke, die Styrol-Butadien-Block- u. -copolymere sowie die Polyisopropene. Auch einsetzbar sind "random (block) polymers", die hergestellt werden durch 1,3-Addition von Butadien oder Isopren an Styrol oder alpha-Methyl Styrol, die Homopolymere oder Copolymere des Ethylens und Propylens, wie der Ethylen-Propylendien-Terpolymere, der natürliche Kautschuk und Norbornen-Polymere wie Polydicyclopentadien. Die Verbindungen aus der Gruppe der Olefinhomopolymere und Copolymere können auch teilhydriert sein.

Selbstverständlich können auch Kombinationen der vorgenannten Haftvermittler in dem erfindungsgemäßen Mittel eingesetzt werden.

Der Haftvermittler bewirkt die Haftung des Mittels an der Oberfläche des Sanitärgegenstandes. Die erfindungsgemäßen Mittel haften sowohl an trockenen als auch an feuchten Oberflächen.

Weiterhin bewirken die speziellen Haftvermittler auch, dass das aufgetragene Mittel an seiner Oberfläche klebrig ist, so dass an die Oberfläche des Haftmittels andere Wirkstoffmittel angeklebt werden können.

Im Allgemeinen bildet der Haftvermittler auch netzwerkartige Strukturen aus, die dem Mittel selbst unter der starken Krafteinwirkung durch Spülwasser die erforderliche Formbeständigkeit verleihen.

Die einzusetzende Konzentration des Haftvermittlers ist von der jeweiligen Stoffklasse und dem Netzwerkbildungsvermögen des Haftvermittlers abhängig und liegt im Allgemeinen zwischen 2 Gew.% und 60 Gew.%, vorzugsweise zwischen 7 Gew.% und 50 Gew.% und besonders bevorzugt zwischen 8 Gew.% und 40 Gew.%.

Weiterhin umfasst das erfindungsgemäße Mittel Füllstoffe, die aus der Gruppe der Tenside, Verdicker, Duftstoffe, Farbstoffe, Salze, Schaumstabilisatoren, Schaumbooster, Schaumgeneratoren und polymeren Naturstoffen ausgewählt sein können.

Als Tenside können prinzipiell alle bekannten anionischen und/oder kationischen und/oder nichtionischen und/oder amphoteren Tenside eingesetzt werden, wobei pulverförmige bis hochpastöse bevorzugt sind. Der Tensidanteil in dem Mittel sollte zwischen 0 Gew.% und 80 Gew.% betragen, vorzugsweise 10 Gew.% bis 60 Gew.% und besonders bevorzugt 25 Gew.% bis 45 Gew.%.

Die anionischen Tenside übernehmen in der vorliegenden Erfindung mehrere Aufgaben; zum einen dienen sie dazu, bei polymeren wasserunlöslichen Matrizes die polymere Matrix zu emulgieren, ohne die Klebefähigkeit komplett zu zerstören. Zum anderen tragen sie wesentlich zur Plastifizierung des Ausgangspolymers (Haftvermittlers) bei, indem sie als Viskositätserhöher (Verdicker) dienen. Vorzugsweise sollten die anionischen Tenside auch stark schäumend sein, um optisch einen Reinigungs-Effekt z.B. in einer Toilettenschüssel zu zeigen. Nicht zuletzt ist eine gute Reinigungswirkung der Tenside erwünscht, die durch die guten Netzeigenschaften unterstützt wird.

Als anionische Tenside werden bevorzugt ein oder mehrere Stoffe aus der Gruppe der Salze der Carbonsäuren, der Schwefelsäurehalbester und der Sulfonsäuren, vorzugsweise aus der Gruppe der Fettsäuren, der Fettalkylschwefelsäuren und der Alkylarylsulfonsäuren, eingesetzt. Üblicherweise liegen die C-Kettenverteilungen der anionischen Tenside im Bereich von 6 bis 40, vorzugsweise 8 bis 30 und insbesondere 12 bis 22 Kohlenstoffatomen.

Carbonsäuren (C6-C22) in Form ihrer Metallsalze (vorzugsweise AlkaliSalze) und deren natürliche oder synthetische Gemische sowie Alkalisalze der Schwefelsäurehalbester und längerkettigen Alkohole können ebenfalls als anionische Tenside eingesetzt werden.

Eine weitere Klasse von anionischen Tensiden, die erfindungsgemäß eingesetzt werden kann, sind die Alkalisalze der Alkyletherschwefelsäuren. Alkyletherschwefelsäuren werden wie die Alkylschwefelsäuren aus Fettalkoholen synthetisiert, welche mit Ethylenoxid zu den betreffenden Fettalkoholethoxylaten umgesetzt werden. Anstelle von Ethylenoxid kann auch Propylenoxid eingesetzt werden. Die nachfolgende Sulfonierung liefert die betreffenden Alkyletherschwefelsäuren.

Auch die Alkalisalze der Alkansulfonsäuren und Olefinsulfonsäuren sind im Rahmen der vorliegenden Erfindung als anionische Tenside einsetzbar. Alkansulfonsäuren können die Sulfonsäuregruppe terminal gebunden (primäre Alkansulfonsäuren) oder entlang der C-Kette enthalten (sekundäre Alkansulfonsäuren). Typische Vertreter sind Alkylbenzolsulfonate, besonders bevorzugt lineare Alkylbenzolsulfonate (LAS).

Die vorstehend genannten anionischen Tenside können in ihrer neutralisierten Form alleine oder in Mischung miteinander eingesetzt werden.

Erfindungsgemäß enthält die Tensidphase bezogen auf ihr Gewicht vorzugsweise 10 bis 90 und besonders bevorzugt 40 bis 85 Gew.% Laurylsulfat.

Als nichtionische Tenside können alkoxylierte, vorzugsweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann bzw. lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, z.B. aus Kokos-, Palm-, Talgfett- oder Oleylalkohol und durchschnittlich 2 bis 8 EO pro Mol Alkohol bevorzugt. Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO.

Als nichtionische Tenside können Alkylglykoside der allgemeinen Formel Alkyl-O(G) eingesetzt werden, wobei Alkyl für einen primären geradkettigen oder methylverzweigten, insbesondere in 2-Stellung methylverzweigten aliphatischen Rest mit 8 bis 22, vorzugsweise 12 bis 18 C-Atomen, steht und G das Symbol für eine Glykosid-einheit mit 5 oder 6 C-Atomen, vorzugsweise für Glucose, ist.

Eine weitere Klasse bevorzugt eingesetzter nichtionischer Tenside, die entweder als alleiniges nichtionisches Tensid oder in Kombination mit anderen nichtionischen Tensiden eingesetzt werden, sind alkoxylierte, vorzugsweise ethoxylierte oder ethoxylierte und propoxylierte Fettsäurealkylester, vorzugsweise mit 1 bis 4 Kohlenstoffatomen in der Alkylkette, insbesondere Fettsäuremethylester. Auch nichtionische Tenside vom Typ der Aminoxide, beispielsweise N-Kokosalkyl-N,N-dimethylaminoxid und N-Talgalkyl-N,N-dihydroxyethylaminoxid oder Alkanolamide können Verwendung finden.

Der Anteil des bzw. der nichtionischen Tenside an der gesamten Tensidphase kann bis zu 50 %, bevorzugt bis zu 30% und besonders bevorzugt bis zu 25% betragen.

Die Tensidphase kann, falls gewünscht, auch mit kationischen oder amphoteren und zwitterionischen Tensiden ausgestattet werden. Beispiel für amphotere Tenside sind Fettsäureamidopropylbetaine mit C5-C21 Fettsäureanteilen, aber auch Amphodiacetate.

Kationische Tenside werden bevorzugt in sauren Formulierungen in Kombination mit bakterizid wirkenden Substanzen eingesetzt. Zwitterionische Tenside können exemplarisch beschrieben werden als quartäre Ammonium-, Phosphonium- oder Sulfonium-Komponenten, die über eine aliphatische-Brücke mit einer weiteren jetzt anionischen Gruppe wie Carboxy, Sulfonat, Sulfat, Phosphat oder Phosphonat verbunden sind.

Als Schäumer können dem Mittel weiterhin Olefinsulfonate, Ethersulfate oder Säuremethyltauride zugesetzt werden. Sollen starke Schäumer Verwendung finden, so können vorzugsweise 1 bis 50, insbesondere 1 - 25 % der Tensidphase durch einen oder mehrere Schäumer z.B. aus der Gruppe der Betaine, der alkoxylierten Alkylethersulfate oder Lactobionsäurederivate ersetzt werden. Diese Schäumer können ausgewählt werden unter den Fettsäureamidopropyl-Betainen mit einem C5 - C21-Fettsäureanteil wie beispielsweise Kokosamidoproplybetain, den Alkali- oder Ammoniumsalzen der Laurylethersulfate mit 1 bis 5 EO, dem Lactobionococoylamid, dem Lactobionooleylamid, dem Lactobionotalgamid etc. oder deren Mischungen. Diese Schäumer lassen sich gut in den Haftkleber einbringen. Vorzugsweise setzt man solche Cotenside ein, die in fester, vorzugsweise pulvriger oder hochviskoser Form vorliegen.

Neben den erfindungsgemäßen Bestandteilen kann das Haftmittel weitere übliche Bestandteile umfassen, beispielsweise Salze, Desinfektionsmittel (z.B. Sauerstoff oder Chlorabspalter), Konservierungsstoffe wie zum Beispiel Isothiazolon-Derivate, Schaumstabilisatoren wie z.B. Alkanolamide, Hydrophobierungsmittel wie z.B. Mineralöle oder (teil)methylierte Siloxane und Silane, Calcium-Dispergatoren wie Natriumsalze von Polycarbonsäuren oder Farbstoffe.

Durch die Zugabe von Parfümöl oder Duftstoffen kann das Haftmittel auch zur Beduftung der Raumluft verwendet werden.

Als Parfümöle bzw. Duftstoffe können einzelne Riechstoffverbindungen, z.B. die synthetischen Produkte der Aldehyde, Alkohole, Ester, Ether, Ketone und Kohlenwasserstoffe eingesetzt werden. Riechstoffverbindungen vom Typ der Ester sind z.B. Benzylacetat oder Benzylformiat. Zu den Ethern zählen beispielsweise Benzylethylether, zu den Aldehyden z.B. Citronellal, zu den Ketonen α-lsomethylionon, zu den Alkoholen Citronellol, Eugenol, Geraniol und Linalool. Zu den Kohlenwasserstoffen gehören hauptsächlich die Terpene wie Limonen und Pinen. Bevorzugt werden jedoch Mischungen verschiedener Riechstoffe verwendet, die gemeinsam die gewünschte Duftnote ergeben. Dazu zählen auch solche Parfümöle, die natürliche Riechstoffgemische enthalten, wie sie aus pflanzlichen Quellen zugänglich sind wie z.B. Pine-, Citrus-, Lavendel-, Minzöl oder Öl aus Orangenschalen.

Die Duftstoffe werden in Konzentrationen zwischen 0,25 Gew.% und 20 %, bevorzugt zwischen 3 Gew.% und 15 Gew.% und besonders bevorzugt zwischen 5 Gew.% und 10 Gew.% direkt in den Ansatz eingearbeitet.

Der Formulierung können - falls gewünscht - auch Salze wie beispielsweise Natriumsulfat als Füllstoffe hinzu gegeben werden, beispielsweise, um die Auflösegeschwindigkeit zu erhöhen. Der Salzanteil kann bei einem besonders kostengünstigen Produkt bis zu 90 Gew.% betragen. Im Allgemeinen liegt der Salzanteil bei bis zu 10 Gew.%, vorzugsweise bei bis zu 5 Gew.%. Geeignete Salze sind Alkalisalze von starken Säuren wie Natriumsulfat, Natriumchlorid oder auch Natriumpolyphosphate. Ebenfalls einsetzbar sind die Alkalisalze der Mono-, Di- und Polycarbonsäuren, aber auch Erdalkalisalze von starken Säuren wie Calciumsulfat oder Salze der Kohlensäure.

Dem Mittel werden zur Erhöhung der Abspülbarkeit Calciumseifen-Dispergatoren wie z.B. Amphodipropionate (Lonza KL Typ) oder Maleinsäure/Acrylsäure-Copolymerisat-Na-Salze (BASF Sokalan Typen wie Sokalan CP5 oder CP45) zugesetzt.

In das Mittel können zudem alle Farbstoffe als Füllstoffe eingearbeitet werden, die keine ausgeprägte Substantivität gegenüber den mit den farbstoffhaltigen Mitteln zu behandelnden Oberflächen haben. Werden wasserlösliche Farbstoffe in der Formulierung eingesetzt und kommt diese dann als Fertigprodukt mit Wasser in Berührung, z.B. beim Bespülen mit Wasser in einer Toilettenschüssel, lassen sich interessante Farbverläufe erzielen, die auch mit Marketing-Argumenten korreliert werden können. Beispielsweise kann eine langsame Blaufärbung (von hellblau nach tiefblau) das langsame Aktivieren eines Wirkstoffes bis zu seiner maximalen Aktivität anzeigen.

Ebenso kann das Haftmittel auch sauer eingestellt sein und beispielsweise Kalk- oder Urinstein lösende Substanzen (Säuren) als Füllstoffe umfassen.

Weiterhin können dem erfindungsgemäßen Mittel (Co)-Verdicker zugegeben werden, um die Plastizität des Mittels zu erhöhen. Als (Co)-Verdicker können beispielsweise Bentonite, Pulvertenside, Xanthane, Polybutadienkautschuke, Polyisopropene, Block-Copolymere, die verknüpfte Oligomere bestehend aus Oligo- oder Polyethylenoxid und/oder Oligo- oder Propylenoxid und/oder Oligo- oder Polybutylenoxid umfassen sowie Arylethoxylate oder Alkyl-Aryl-Ethoxylate verwendet werden. Auch die polymeren Naturstoffe wie die Lignine oder deren Alkali- oder Erdalkalisalze können als (Co)-Verdicker verwendet werden.

Eine bevorzugte Gruppe von (Co)-Verdickern sind die hydrophilen Xanthane. Durch ihren Einsatz wird eine sehr hydrophile Verbindung in das Mittel eingeführt, die beim Ankleben des Mittels an eine feuchte Oberfläche das Wasser umgehend "aufsaugt".

Weiterhin können dem Haftmittel Hydrophöbierer wie beispielsweise Aerosil, insbesondere durchmethyliertes Aerosil (Fa. Carbot Carbon) als Füllstoffe zugegeben werden.

Falls eine Beschleunigung des Haftens gewünscht sein sollte, können dem Mittel auch sogenannte Tackifier, insbesondere aus der Klasse der Kohlenwasserstoffharze, der Naturharze wie Tallharz oder Balsamharz oder der Polyterpenharze, als Füllstoffe zugegeben werden.

Um unangenehme Gerüche zu binden kann das Haftmittel weiterhin Geruchsbinder (malodor counteractants), die vorzugsweise den Duftstoffen zugegeben werden, enthalten. Solche Geruchsbinder sind beispielsweise in der US 7,288,507 B2 beschrieben.

Das erfindungsgemäße Haftmittel kann in hygienischer Weise ohne Berührung von mit dem WC-Becken verbundenen, möglicherweise verunreinigten Vorrichtungen aufgebracht und erneuert werden.

Ein wesentlicher Vorteil des erfindungsgemäßen Mittels besteht darin, dass es nach den Wünschen des Verbrauchers portionierbar ist und/oder in unterschiedlichen Portionspackungen ausgelobt werden kann. Die Applikation des Haftmittel kann beispielsweise mittels einer Applikationsspritze erfolgen oder durch Applikation vorportionierter Mengen mittels geeigneter Vorrichtungen. Bei diesen Applikationsvorrichtungen kann es sich beispielsweise um "Klammersysteme", Greifer oder von Folien abziehbare Plättchen, Spendersysteme mit vorgespannten Elementen, die eine entsprechende Portion z.B. an eine keramische Oberfläche anschießen, handeln.

Das erfindungsgemäße Mittel kann auch auf einfache Weise gleichzeitig an verschiedenen Stellen des Sanitärgegenstands appliziert werden, beispielsweise, um mehrere Wirkstoffmittel, die sich in ihrer Wirkung in direkter Nähe beeinträchtigen würden wie beispielsweise eine Beduftungstablette und eine Bleichmitteltablette, anzukleben.

Die erreichte Haftung ist an dem Sanitärgegenstand selbst bei einer Anbringung an einer senkrechten Fläche so gut, dass sich das Mittel sogar unter der zusätzlichen Krafteinwirkung von Spülwasserströmen nicht ablöst.

Die erfindungsgemäßen Haftmittel sind erst nach einer größeren Anzahl von Spülvorgängen abspülbar. Die Anzahl der Spülvorgänge richtet sich natürlich nach der Zusammensetzung des jeweiligen Mittels, der aufgebrachten Menge sowie der Geometrie des aufgebrachten Mittels und liegt im Allgemeinen bei einem Auftrag mit einer Dicke von 2 bis 5 mm zwischen 50 und 150, insbesondere mehr als 120 Spülungen.

Wird das Mittel als Kleber in der Toilettenschüssel verwendet, ist die Haltbarkeit im Wesentlichen definiert über die Abspülzeit des aufgebrachten weiteren Körpers (stückförmigen Mittels). Im Falle eines herkömmlichen Toilettensteines kann damit eine Standzeit von 100 bis 200, in manchen Fällen >250 Spülungen erreicht werden, wobei das Haftklebemittel sukzessive zusammen mit dem angeklebten Material abgewaschen wird. Die aufgetragene Menge beträgt 3 bis 15 Gew.%, insbesondere 5 bis 10 Gew.%, der Masse des aufgeklebten Mittels. Vorzugsweise ist das erfindungsgemäße Mittel weiß, salbenartig, pastös und/oder cremeartig und formstabil, so dass es nicht "herunterläuft" oder "tropft".

Die Haftung und auch die Form der Mittel bleiben trotz der durch die Wasserspülung einwirkenden erheblichen Kräfte (Reibung, Deformation, Scherwirkung) erhalten.

Das Mittel zeigt im Wesentlichen Strukturviskosität, d.h. die Viskosität nimmt bei steigenden Scherkräften ab. Sind die Scherraten gering, ist allerdings eine starke Fließbehinderung zu beobachten; außerdem zeigen die Viskositätskurven bei einer Scherratenrampe zwischen 2,5 s⁻¹ und 30 s⁻¹ das Auftreten von lokalen Maxima. Offensichtlich gibt es unterschiedlich viskose Bereiche oder das Mittel verändert seine Struktur über die kurze Messzeit (Rampenzeit 100 sec.).

Die an einem Haake-Viskosimeter, System Platte/Platte, Plattendurchmesser 10 mm bei einem Schergefälle von 2,62 s⁻¹ und 20°C zu bestimmenden Viskositäten dieser Mittel sollten wenigstens 30 Pa s, vorzugsweise wenigstens 45 und besonders bevorzugt wenigstens 100 Pa s betragen. Vorzugsweise sollten die Viskositäten zwischen 150 beziehungsweise 300 und 6000 Pa s liegen und besonders bevorzugt zwischen 200 und 1000 beziehungsweise zwischen 1000 und 4000 Pa s.

Vorzugsweise geben die erfindungsgemäßen Mittel, die Tenside umfassen, einen feinblasigen Schaum, der durch geeignete Zusätze (Schaumbooster) in seinem Volumen eingestellt werden kann. Die Schaumzahlen der erfindungsgemäßen Mittel sollten über 40 ml Schaum liegen. Besonders bevorzugt sind Mittel mit Schaumzahlen > 60 ml, ganz besonders bevorzugt sind Schaumzahlen von 140 ml oder gar mehr als 200 ml.

Die Oberflächenspannung der Mittel kann zwischen 50 und 65 mN/m liegen. Bevorzugt sind solche Mittel, deren Oberflächenspannung <= 60m N/m beträgt. Besonders bevorzugt sind Mittel, die Oberflächenspannungen gleich oder kleiner als 40 mN/m erreichen. Die Oberflächenspannung ist ein Maß für die Benetzung der Oberfläche. Je kleiner die Oberflächenspannung ist, umso besser wird die Oberfläche benetzt. Eine gute Netzwirkung ist Voraussetzung für eine gute Reinigungsleistung der betrachteten Mittel.

Die Herstellung des erfindungsgemäßen Mittels erfolgt durch Zusammenrühren der Komponenten bei Raumtemperatur.

Die Erfindung wird nachfolgend anhand von verschiedenen Ausführungsbeispielen und Versuchen beschrieben.

In **Tabelle 1**, die am Ende der Beschreibung angefügt ist, sind verschiedene Rezepturen der erfindungsgemäßen Haftmittel zusammengestellt.

Die Spülzahlen in Tabelle 1 wurden an aufgetragenen Mengen von 2 - 5 g bestimmt.

In **Tabelle 2**, die weiterhin am Ende der Beschreibung angefügt ist, sind die zur Herstellung der erfindungsgemäßen Mittel in Tabelle 1 verwendeten Edukte aufgelistet.

Die erfindungsgemäßen Haftmittel gemäß den Rezepturen V18 und V24 zeigen eine starke Haftung und sind an ihrer Außenseite so klebrig, dass an den Mitteln herkömmliche Toilettenreinigungsmittel mit einer Masse von bis zu 50 g angeklebt werden können, je nachdem, welche Mengen an Klebstoff aufgetragen werden. Das Verhältnis von angeklebter Masse der stückförmigen Mittel zur Klebstoffmasse liegt dabei maximal bei 100: 1, bevorzugt bei maximal 50:1 und besonders bevorzugt bei maximal 10:1. Natürlich wird eine bessere Haftung des Mittels erzielt, wenn die Klebefläche größer ist. Die üblichen Klebeflächen betragen zwischen 1000 mm² und 800 mm² (vollflächiger Kontakt). Sie können aber auch weniger als 400 mm² betragen, wenn der Klebstoff in Form von umlaufenden Raupen aufgetragen wird.

In allen abgespülten Versuchen wurden als Tenside anionische Tenside eingesetzt. Die Spülzahlen dieser Mittel zeigen relativ hohe bis hohe Standzeiten an.

Die Rezeptur V24 umfasst nur ein (anionisches) Tensid und einen Haftvermittler. Auch dieses nur aus zwei Komponenten bestehende Mittel zeigt das gewünschte Haftvermögen, die erforderliche Klebrigkeit und Spülzahlen von mehr als 100.

Der reine auf die Toilettenschüssel aufgetragene Haftvermittler Versagel M1600 (hydriertes Butylen/Ethylen/Styrolcopolymer) hingegen klebt in der Schüssel fest und lässt sich auch nach einer Vielzahl von Spülzyklen überhaupt nicht abspülen. An diesen Haftvermittler können auch Toilettensteine angeklebt werden, allerdings wandern die Toilettensteine mit dem Klebemittel Versagel nach und nach nach unten.

Die Tabelle 3 zeigt, dass die erfindungsgemäßen Mittel sich signifikant von den bisher bekannten Mitteln der EP 1325103 B1 unterscheiden.

Zur Bestimmung der Schaumzahlen überführt man 100 ml der auf 20 °C temperierten Stammlösung in einen 250 ml Mischzylinder und verschließt diese mit einem PTFE-Stopfen. Dann wird der Zylinder zwanzigmal hin und her gewendet (20 mal auf den Kopf gestellt). Nach jeweils 30 sec/5 min/30 min wird das erzeugte Schaumvolumen (mi) abgelesen und notiert.

Alle untersuchten erfindungsgemäßen Mittel haften/kleben hervorragend sowohl an trockenen als auch an nassen Oberflächen.

Auch sind sie an ihrer Außenseite so klebrig, dass an den Mitteln herkömmliche Toilettenreinigungsmittel mit einer Masse von bis zu 50 g oder gar mehr angeklebt werden können, je nachdem, welche Mengen an Klebstoff aufgetragen werden. In allen abgespülten Versuchen wurden als Tenside anionische Tenside eingesetzt. Die Spülzahlen dieser Mittel zeigen relativ hohe bis hohe Standzeiten an.

**Tabelle 1**

| | **v18** | **v24** | |
|---|---|---|---|
| | **in g** | **in g** | |
| Tensopol USP 94 | 49,6 | 70,0 | **Tensid** |
| Versagel M1600 | 36,7 | 30,0 | **Haftvermittler** |
| Kelzan ASX | 0,99 | | **Verdicker** |
| Orange Fun | 12,71 | | **Parfüm** |
| Summe [in g] | 100,00 | 100 | |
| | | | |
| Spülungen: | > 150 | 130 | |

**Tabelle 2**

| | Hersteller | **Typ** | **Chemie** | **Funktion** |
|---|---|---|---|---|
| Tensopol USP 94 | Manro | Tensopol USP 94 | C12-C16 Laurylsulfat | Tensid |
| Orange Fun | Quest | # FS61415 | | Parfüm |
| Kelzan ASX | Kelco | Kelzan ASX | Xanthan | Verdicker/ Coverdicker |
| Versagel M1600 | sblack/Penreco | M1600 | Mineral oll (and) Hydrogenated Butylene/Ethylene/Styrene Copolymer (and) Hydrogenated Ethylen/Propylen/Styrene Copolymer | Haftvermittler |

**Tabelle 3**

| **Versuchs Nummer** | **Schaumzahl [mm]** Schaum [ml]; (0,1%); | **[Pas], PP, 1mm, 20°C, γ'=2,62 s⁻** |
|---|---|---|
| 18 | n.b. | 328 |
| Vergleichsgel aus EP 325103 B1 | 70/25/10 | 524 |

## Patentansprüche

1. Mittel für den Sanitärbereich, welches Mittel unmittelbar auf dem Sanitärgegenstand applizierbar ist, dort haftet und erst nach einer größeren Anzahl von Spülvorgängen abspülbar ist, wobei das Mittel Füllstoffe aus der Gruppe der Tenside enthält sowie einen Haftvermittler, **dadurch gekennzeichnet, dass** der Haftvermittler ausgewählt wird aus der Gruppe der hydrierten Polystyrolderivate, der Olefinhomopolymere und der Copolymere von zwei oder mehr Olefinen, wobei die Olefinhomopolymere und -copolymere auch teilhydriert sein können und die Viskosität des Mittels wenigstens 30 Pas, gemessen mit einem Haake-Viskosimeter, System Platte/Platte, Plattendurchmesser 10 mm, bei einem Schergefälle von 2,62 s⁻¹ und 20 °C beträgt und das Mittel so klebrig ist, dass es zur Befestigung von stückförmigen Mitteln in der Toilettenschüssel dienen kann.

2. Mittel nach Anspruch 1, **dadurch gekennzeichnet, dass** die Haftvermittler aus der Gruppe der Polystyrolderivate in Mineralöl gelöste, quervernetzte Polystyrolderivate, vorzugsweise Alkylenstyrol-Copolymere, insbesondere aus der Gruppe der hydrierten Butylen/Ethylen/Styrol-Copolymere und der hydrierten Ethylen/Propylen/Styrol-Copolymere, sind.

3. Mittel nach Anspruch 1, **dadurch gekennzeichnet, dass** die Haftvermittler aus der Gruppe der nicht wasserlöslichen Olefinhomopolymere und der Copolymere von zwei und mehr Oleflnen ausgewählt werden aus der Gruppe der Polybutadienkautschuke, der Styrol-Butadien-Block- u. - copolymere, der Polyisopropene, der random (block) polymere, die hergestellt werden durch 1,3-Addition von Butadien oder Isopren an Styrol oder alpha-Methyl Styrol, der Homopolymere oder Copolymere des Ethylens und Propylens wie der Ethylen-Propylendien-Terpolymere, des natürlichen Kautschuks und der Norbornen-Polymere wie des Polydicyclopentadiens.

4. Mittel nach Anspruch 1, **dadurch gekennzeichnet, dass** die Konzentration des Haftvermittlers in dem Mittel zwischen 2 Gew.% und 60 Gew.%, vorzugsweise zwischen 7 und 50 Gew.% und besonders bevorzugt zwischen 8 Gew.% und 40 Gew.% beträgt.

5. Mittel nach einem der Ansprüche 1 bis 3, **dadurch gekennzeichnet, dass** die Konzentration des Haftvermittlers in dem Mittel zwischen 15 Gew.% und 80 Gew.%, vorzugsweise zwischen 20 und 70 Gew.% und besonders bevorzugt zwischen 30 Gew.% und 50 Gew.% beträgt.

6. Mittel nach einem der vorangegangenen Ansprüche, **dadurch gekennzeichnet, dass** die Füllstoffe aus der Gruppe der Verdicker, Duftstoffe, Farbstoffe, Salze, Schaumstabilisatoren, Schaumbooster, Schaumgeneratoren und polymeren Naturstoffen ausgewählt werden.

7. Mittel nach Anspruch 1, **dadurch gekennzeichnet, dass** der Tensidanteil in dem Mittel bis zu 80 Gew.%, vorzugsweise 10 bis 60 Gew.% und besonders bevorzugt 25 bis 45 Gew.% beträgt.

8. Mittel nach einem der Ansprüche 1 oder 7, **dadurch gekennzeichnet, dass** die Tenside pulverförmig oder hochpastös sind.

9. Mittel nach einem der Ansprüche 1 bis 8, **dadurch gekennzeichnet, dass** die Tenside anionische Tenside sind und ausgewählt werden aus der Gruppe der Salze der Carbonsäuren, der Schwefelsäurehalbester, der Sulfonsäuren, der längerkettigen Alkohole und der Fettalkoholethoxylaten.

10. Mittel nach einem der Ansprüche 1 oder 6 bis 9, **dadurch gekennzeichnet, dass** die Tenside nichtionische Tenside sind und aus der Gruppe der Alkoholethoxylate, der Alkylglykoside, der alkoxylierten Fettsäurealkylester, der Aminoxide und der Alkanolamide ausgewählt werden.

11. Mittel nach Anspruch 6, **dadurch gekennzeichnet, dass** das Mittel Duftstoffe bzw. Parfümöle in einer Konzentrationen zwischen 0,25 Gew.% und 20 Gew.%, bevorzugt zwischen 3 Gew.% und 15 Gew.% und besonders bevorzugt zwischen 5 Gew.% und 10 Gew.% enthält.

12. Mittel nach Anspruch 6, **dadurch gekennzeichnet, dass** das Mittel bis zu 90 Gew.% Salze, insbesondere bis zu 10 Gew.%, vorzugsweise bis zu 5 Gew.% Salze, vorzugsweise aus der Gruppe der Alkali- und Erdalkalisalze der starken Säuren oder der Mono-, Di- und Polycarbonsäuren, enthält.

13. Mittel nach Anspruch 6, **dadurch gekennzeichnet, dass** das Mittel (Co)-Verdicker aus der Gruppe der Bentonite, Pulvertenside, Xanthane, Polybutadienkautschuke, Polyisopropene, Block-Copolymere, Arylethoxylate oder Alkyl-Aryl-Ethoxylate oder polymere Naturstoffe enthält.

14. Mittel nach einem der vorangegangenen Ansprüche, **dadurch gekennzeichnet, dass** das Mittel ein wasserlöslicher und/oder wasserdispergierbarer temporär haftender Klebstoff ist.

15. Verwendung eines Mittels nach einem der vorangegangenen Ansprüche, **dadurch gekennzeichnet, dass** die anklebbaren stückförmigen Mittel herkömmliche Rlmblocks mit einer oder mehreren Phasen, Rimblocks mit Duftphase, Rimblocks mit Bleichmittel, gepresste Tabletten, Wirkstoffe enthaltende wasserlösliche oder wasserunlösliche Kunststoffe, Beduftungstabletten, feste Beduftungsgele, Bleichmitteltabletten, Entkalkungstabletten oder Intensivreinigungstabletten sind.

16. Mittel nach einem der vorangegangenen Ansprüche, **dadurch gekennzeichnet, dass** das Mittel reinigende und/oder duftende und/oder bleichende und/oder färbende Zusätze enthält.

17. Mittel nach einem der vorangegangenen Ansprüche, **dadurch gekennzeichnet, dass** das Mittel salbenartig, pastös und/oder cremeartig und formstabil ist.

18. Mittel nach einem der vorangegangenen Ansprüche, **dadurch gekennzeichnet, dass** die Oberflächenspannung des Mittels zwischen 50 und 65 mN/m, insbesondere unter 60 mN/m, beträgt.

19. Toilettenreinigungsset umfassend wenigstens ein Mittel nach einem der vorangegangenen Ansprüche und ein oder mehrere stückförmige Mittel aus der Gruppe der Rimblocks mit einer oder mehreren Phasen, Rimblocks mit Duftphase, Rimblocks mit Bleichmittel, der gepressten Tabletten, der Wirkstoffe enthaltenden wasserlöslichen oder wasserunlöslichen Kunststoffe, der Beduftungstabletten, der festen Beduftungsgelen, der Bleichmitteltabletten, der Entkalkungstabletten oder der Intensivreinigungstabletten.

20. Verwendung eines Mittels nach einem der vorangegangenen Ansprüche als Kaltkleber im Sanitärbereich, insbesondere zum Befestigen von Gegenständen in Urinalen, Pissoirs, Handspülbecken oder Kacheln, oder in Küchen, Restaurants, Schlachthäusern, Waschanlagen oder zur Applikation an/in Abflüssen oder Gullis oder als Paste zur Aufnahme von Ködern gegen Ungeziefer oder zur Befestigung an Scheiben oder Fassaden.

21. Verwendung nach Anspruch 20, **dadurch gekennzeichnet, dass** die Masse des auf den Gegenstand aufgetragenen Mittels 3 bis 15 Gew.% der Masse des Gegenstandes beträgt.

22. Verfahren zur Herstellung eines Mittels nach einem der vorangegangenen Ansprüche, **dadurch gekennzeichnet, dass** die einzelnen Komponenten bei Raumtemperatur zusammen gerührt werden.

## Claims

1. An agent for the sanitary sector, which agent can be applied directly on the sanitary object, adheres there and can be flushed away only after a relatively large number of flushing operations, where the agent comprises fillers from the group of surfactants and also an adhesion promoter, **characterized in that** the adhesion promoter is selected from the group of hydrogenated polystyrene derivatives and olefin homopolymers and copolymers of two or more olefins, where the olefin homopolymers and copolymers may also be partially hydrogenated, and the viscosity of the agent is at least 30 Pas, measured using a Haake viscometer, plate/plate system, plate diameter 10 mm, at a shear gradient of 2,62 s⁻¹ and 20°C and the agent is so sticky that it can serve to attach bar-shaped agents in the toilet bowl.

2. The agent as claimed in claim 1, **characterized in that** the adhesion promoters from the group of polystyrene derivatives are crosslinked polystyrene derivatives dissolved in mineral oil, preferably alkylene styrene copolymers, in particular from the group of hydrogenated butylene/ethylene/styrene copolymers and hydrogenated ethylene/propylene/styrene copolymers.

3. The agent as claimed in claim 1, **characterized in that** the adhesion promoter from the group of non-water-soluble olefin homopolymers and the copolymers from two or more olefins are selected from the group of polybutadiene rubbers, styrene-butadiene block polymers and copolymers, polyisoprenes, random (block) polymers which are prepared by 1,3-addition of butadiene or isoprene onto styrene or alpha-methylstyrene, homopolymers or copolymers of ethylene and propylene, such as ethylene-propylenediene terpolymers, natural rubber and norbornene polymers such as polydicyclopentadiene.

4. The agent as claimed in claim 1, **characterized in that** the concentration of the adhesion promoter in the agent is between 2% by weight and 60% by weight, preferably between 7 and 50% by weight and particularly preferably between 8% by weight and 40% by weight.

5. The agent as claimed in one of claims 1 to 3, **characterized in that** the concentration of the adhesion promoter in the agent is between 15% by weight and 80% by weight, preferably between 20 and 70% by weight and particularly preferably between 30% by weight and 50% by weight.

6. The agent as claimed in one of the preceding claims, **characterized in that** the fillers are selected from the group of thickeners, fragrances, dyes, salts, foam stabilizers, foam boosters, foam generators and polymeric natural substances.

7. The agent as claimed in claim 1, **characterized in that** the surfactant fraction in the agent is between 0 and 80% by weight, preferably 10 to 60% by weight and particularly preferably 25 to 45% by weight.

8. The agent as claimed in one of claims 1 or 7, **characterized in that** the surfactants are pulverulent or highly pasty.

9. The agent as claimed in one of claims 1 to 8, **characterized in that** the surfactants are anionic surfactants and are selected from the group of salts of carboxylic acids, sulfuric acid half-esters, sulfonic acids, relatively long-chain alcohols and fatty alcohol ethoxylates.

10. The agent as claimed in one of claims 1 or 6 to 9, **characterized in that** the surfactants are nonionic surfactants and are selected from the group of alcohol ethoxylates, alkyl glycosides, alkoxylated fatty acid alkyl esters, amine oxides and alkanolamides.

11. The agent as claimed in claim 1, **characterized in that** the agent comprises fragrances or perfume oils in a concentrations between 0,25% by weight and 20% by weight, preferably between 3% by weight and 15% by weight and particularly preferably between 5% by weight and 10% by weight.

12. The agent as claimed in claim 6, **characterized in that** the agent comprises up to 90% by weight of salts, in particular up to 10% by weight, preferably up to 5% by weight, of salts, preferably from the group of alkali metal and alkaline earth metal salts of strong acids or of mono-, di- and polycarboxylic acids.

13. The agent as claimed in claim 6, **characterized in that** the agent comprises (co)thickeners from the group of bentonites, powder surfactants, xanthans, polybutadiene rubbers, polyisopropenes, block copolymers, aryl ethoxylates or alkyl-aryl ethoxylates or polymeric natural substances.

14. The agent as claimed in one of the preceding claims, **characterized in that** the agent is a water-soluble and/or water-dispersible temporarily adhering adhesive.

15. The use of an agent as claimed in one of the preceding claims, **characterized in that** the bar-shaped compositions that can be stuck on are conventional rim blocks with one or more phases, rim blocks with scented phase, rim blocks containing bleach, compacted tablets, water-soluble or water-insoluble plastics comprising active ingredients, deodorization tablets, solid deodorization gels, bleaching tablets, descaling tablets or intensive cleaning tablets.

16. The agent as claimed in one of the preceding claims, **characterized in that** the agent comprises cleaning and/or deodorization and/or bleaching and/or coloring additives.

17. The agent as claimed in one of the preceding claims, **characterized in that** the agent is ointment-like, pasty and/or cream-like and dimensionally stable.

18. The agent as claimed in one of the preceding claims, **characterized in that** the surface tension of the agent is between 50 and 65 mN/m, in particular below 60 mN/m.

19. A toilet cleaning set comprising at least one agent as claimed in one of the preceding claims and one or more bar-shaped compositions from the group of rim blocks having one or more phases, rim blocks with scented phase, rim blocks containing bleach, compacted tablets, water-soluble or water-insoluble plastics comprising active ingredients, deodorization tablets, solid deodorization gels, bleaching tablets, descaling tablets or intensive cleaning tablets.

20. The use of an agent as claimed in one of the preceding claims as cold adhesive in the sanitary sector, in particular for attaching objects in urinals, handwashing basins or tiles, or in kitchens, restaurants, slaughter houses, washing facilities or for application on/in drains or gullies or as paste for accommodating bait to combat vermin or for attaching to windows or facades.

21. The use as claimed in claim 20, **characterized in that** the mass of the agent applied to the object is 3 to 15% by weight of the mass of the object.

22. A method for producing an agent as claimed in one of the preceding claims, **characterized in that** the individual components are stirred together at room temperature.

## Revendications

1. Agent pour le domaine sanitaire, lequel agent peut être appliqué directement sur l'objet sanitaire, y adhère et ne peut être éliminé par rinçage qu'après un grand nombre de cycles de rinçage, l'agent contenant des charges du groupe des agents tensioactifs ainsi qu'un promoteur d'adhérence, **caractérisé en ce que** le promoteur d'adhérence est choisi dans le groupe des dérivés de polystyrène hydrogénés, des homopolymères d'oléfines et des copolymères de deux oléfines ou plus, les homopolymères d'oléfines et les copolymères d'oléfines pouvant être aussi partiellement hydrogénés et la viscosité de l'agent est d'au moins 30 Pa.s, mesurée avec un viscosimètre de Haake, système plaque/plaque, diamètre des plaques 10 mm, à un gradient de cisaillement de 2,62 s⁻¹ et à 20°C, et l'agent présente une adhésivité telle qu'il peut servir à la fixation d'agents en forme de morceaux dans la cuvette des toilettes.

2. Agent selon la revendication 1, **caractérisé en ce que** les promoteurs d'adhérence du groupe des dérivés de polystyrène sont des dérivés de polystyrène réticulés transversalement et dissous dans de l'huile minérale, de préférence des copolymères d'alkylène-styrène, en particulier du groupe des copolymères butylène-éthylène-styrène hydrogénés et des copolymères éthylène-propylène-styrène hydrogénés.

3. Agent selon la revendication 1, **caractérisé en ce que** les promoteurs d'adhérence sont choisis dans le groupe des homopolymères d'oléfines non solubles dans l'eau et des copolymères de deux oléfines ou plus, du groupe des caoutchoucs de polybutadiène, des polymères/copolymères à blocs de styrène-butadiène, des polyisopropylènes, des polymères (à blocs) statistiques qui sont préparés par 1,3-addition de butadiène ou d'isoprène sur du styrène ou de l'alpha-méthylstyrène, des homopolymères ou copolymères de l'éthylène et du propylène, tels que les terpolymères d'éthylène-propylène-diène, du caoutchouc naturel et des polymères de norbornène tel que du polydicyclopentadiène.

4. Agent selon la revendication 1, **caractérisé en ce que** la concentration en promoteur d'adhérence dans l'agent est comprise entre 2 et 60% en poids, de préférence entre 7 et 50% en poids et de manière particulièrement préférée entre 8 et 40% en poids.

5. Agent selon l'une quelconque des revendications 1 à 3, **caractérisé en ce que** la concentration en promoteur d'adhérence dans l'agent est comprise entre 15 et 80% en poids, de préférence entre 20 et 70% en poids et de manière particulièrement préférée entre 30 et 50% en poids.

6. Agent selon quelconque l'une des revendications précédentes, **caractérisé en ce que** les charges sont choisies dans le groupe des épaississants, des substances odoriférantes, des colorants, des sels, des stabilisateurs de mousse, des boosters de mousse, des générateurs de mousse et des substances naturelles polymères.

7. Agent selon la revendication 1, **caractérisé en ce que** la proportion d'agent tensioactif dans l'agent est de jusqu'à 80% en poids, de préférence de 10 à 60% en poids, et de manière particulièrement préférée de 25 à 45% en poids.

8. Agent selon l'une quelconque des revendications 1 ou 7, **caractérisé en ce que** les agents tensioactifs sont sous forme de poudre ou hautement pâteux.

9. Agent selon l'une quelconque des revendications 1 à 8, **caractérisé en ce que** les agents tensioactifs sont des agents tensioactifs anioniques et choisis dans le groupe des sels des acides carboxyliques, des semi-esters de l'acide sulfurique, des acides sulfoniques, des alcools à longue chaîne et des éthoxylates d'alcools gras.

10. Agent selon l'une quelconque des revendications 1 ou 6 à 9, **caractérisé en ce que** les agents tensioactifs sont des agents tensioactifs non ioniques et choisis dans le groupe des éthoxylates d'alcool, des alkylglycosides, des esters alkyliques d'acides gras alcoxylés, des oxydes d'amine et des alcanolamides.

11. Agent selon la revendication 6, **caractérisé en ce que** l'agent contient des substances odoriférantes ou des huiles parfumées en une concentration entre 0,25% en poids et 20% en poids, de préférence entre 3% en poids et 15% en poids et de manière particulièrement préférée entre 5% en poids et 10% en poids.

12. Agent selon la revendication 6, **caractérisé en ce que** l'agent contient jusqu'à 90% en poids de sels, en particulier jusqu'à 10% en poids, de préférence jusqu'à 5% en poids de sels, de préférence du groupe des sels de métal alcalin et alcalino-terreux des acides forts ou des acides monocarboxyliques, dicarboxyliques et polycarboxyliques.

13. Agent selon la revendication 6, **caractérisé en ce que** l'agent contient des (co)-épaississants du groupe des bentonites, des agents tensioactifs sous forme de poudre, des xanthanes, des caoutchoucs de polybutadiène, des polyisopropylènes, des copolymères à blocs, des aryléthoxylates ou des alkyl-aryléthoxylates ou des substances naturelles polymères.

14. Agent selon quelconque l'une des revendications précédentes, **caractérisé en ce que** l'agent est un adhésif à adhérence temporaire soluble dans l'eau et/ou dispersible dans l'eau.

15. Utilisation d'un agent selon l'une quelconque des revendications précédentes, **caractérisée en ce que** les agents à coller en morceaux sont des blocs usuels présentant une ou plusieurs phases, des blocs avec une phase odorante, des blocs avec un agent de blanchiment, des comprimés pressés, des matériaux synthétiques solubles ou insolubles dans l'eau contenant des substances actives, des comprimés parfumants, des gels solides parfumants, des comprimés d'agent de blanchiment, des comprimés détartrants ou des comprimés à nettoyage intensif.

16. Agent selon quelconque l'une des revendications précédentes, **caractérisé en ce que** l'agent contient des additifs de nettoyage et/ou qui sentent bon et/ou qui blanchissent et/ou qui colorent.

17. Agent selon quelconque l'une des revendications précédentes, **caractérisé en ce que** l'agent est de type onguent, pâte et/ou crème et de forme stable.

18. Agent selon quelconque l'une des revendications précédentes, **caractérisé en ce que** la tension superficielle de l'agent est comprise entre 50 et 65 mN/m, en particulier inférieure à 60 mN/m.

19. Set de nettoyage de toilettes, comprenant au moins un agent selon l'une quelconque des revendications précédentes et un ou plusieurs agents en morceau, du groupe des blocs WC présentant une ou plusieurs phases, des blocs WC avec une phase odorante, des blocs WC avec un agent de blanchiment, des comprimés pressés, des matériaux synthétiques solubles ou insolubles dans l'eau contenant des substances actives, des comprimés qui sentent bon, des gels solides qui sentent bon, des comprimés d'agent de blanchiment, des comprimés détartrants ou des comprimés à nettoyage intensif.

20. Utilisation d'un agent selon l'une quelconque des revendications précédente comme colle froide dans le domaine sanitaire, en particulier pour la fixation d'objets dans des urinoirs, des vespasiennes, des éviers ou des faïences ou dans les cuisines, les restaurants, les abattoirs, les installations de lavage ou pour l'application sur/dans des écoulements ou des égouts ou comme pâte pour absorber des appâts contre la vermine ou pour la fixation sur des vitres ou des façades.

21. Utilisation selon la revendication 20, **caractérisée en ce que** la masse de l'agent appliqué sur l'objet représente 3 à 15% en poids de la masse de l'objet.

22. Procédé pour la préparation d'un agent selon l'une quelconque des revendications précédentes, **caractérisé en ce que** les différents composants sont brassés les uns avec les autres à température ambiante.
